# EUROPEAN PATENT APPLICATION

(11) **EP 1 101 491 A2**
(43) Date of publication of application: **23.05.2001**
(21) Application number: 99931264.8
(22) Date of filing: 21.07.1999
(51) Int. Cl.: A61K 9/48, A61K 35/78

(54) **SOFT GELATINE CAPSULES CONTAINING OLIVE OIL**

(30) Priority: 21.07.1998 ES 9801543
(71) Applicant: Biocosmetics, S.L., 28033 Madrid (ES)
(72) Inventor: ALVAREZ HERNANDEZ, Maria, E-28023 Madrid (ES)
(74) Representative: Carpintero Lopez, Francisco
(86) International application number: ES9900233
(87) International publication number: WO0004874

(57) **Abstract**

The capsules consist of a gelatinous wall comprising a mixture of gelatine, water and a plasticiser, and olive oil, optionally along with other interesting additives and substances. These capsules are suitable for nutritional, cosmetic and/or therapeutic applications.

## Description

### FIELD OF THE INVENTION

This invention relates to a new manner of administering olive oil, specifically in the form of soft gelatine capsules containing olive oil, suitable for nutritional, cosmetic and/or therapeutic applications.

### BACKGROUND OF THE INVENTION

Olive oil, obtained by crushing olives, the fruit of the olive tree (*Oleus europaea* L.], approximately consists of an 84% mixture of several glycerides of unsaturated fatty acids, mainly oleic acid (between 70 and 80% of the total) and is commonly used for foods purposes (salads, preparing food products, etc.), although other therapeutic and cosmetic applications are known to exist.

Oleic acid [*cis*-9-octadecenoic acid] is a monounsaturated fatty acid which is seemingly responsible for many beneficial effects of olive oil. It is well-known that oleic acid helps prevent arteriosclerosis, increases the level of cholesterol attached to high-density lipoproteins (HDL) and reduces the level of cholesterol attached to low-density lipoproteins (LDL), thereby providing a suitable manner of checking the appearance of cardiovascular diseases.

Furthermore, olive oil contains both vitamins A, E, F and K and polyphenols, and it is believed that such antioxidant substances as the vitamins (A and E) and polyphenols present in olive oil provide the organism with a defence mechanism which delays ageing, prevents arteriosclerosis, the appearance of breast cancer, hepatic disorders and inflammation.

Olive oil is well tolerated in the stomach, has beneficial effects on gastritis and ulcers, is cholagogic, stimulates the secretion of pancreatic hormones and bile, and reduces the effects of cholelithiasis. On the other hand, its excellent digestibility results in a full absorption of nutrients, especially vitamins and mineral salts, and contributes the necessary oleates for the bone system. The mineralising effect of olive oil is excellent in both childhood and old age, at which stage there are usually mineralisation problems.

Olive oil also has a beneficial effect on the brain and the central nervous system, protecting the body from the appearance of infections and helping inner and outer tissue heal.

In addition to the above-mentioned therapeutic benefits, olive oil has cosmetic applications and is now starting to be used as a skin protector, preventing the appearance of wrinkles, and as a reliever in dry and scaly skin. It is also used to restore the brightness and strength of strained hair and eyebrows and restore the vitality of frail hair.

Olive oil is currently taken orally in the form of an oral liquid or else with food, mixed with other food products as such or processed. Taking olive oil in liquid oral form has a number of disadvantages, such as:
- a container for the oil and a spoon have to be used, wherefore it is inconvenient and scarcely practical to administer, causing the user a bother in regard to handling and carrying the same;
- its dosage is rather inaccurate;
- the taste of olive oil in liquid oral form is very peculiar and poses acceptance problems by a great many people; and
- administering olive oil in liquid oral form predisposes against taking the same, for it encourages vomiting and retching, which means that consuming it thus tends to be avoided and its beneficial effects on the organism are consequently lost.

There is hence a need to develop new forms of administering olive oil which may overcome the above-mentioned drawbacks.

Many tests made have shown that not all forms of administering olive oil orally are suitable because administering olive oil in sublingual preparations is not feasible.

The present invention provides a solution to the existing need consisting of developing a new form of administering olive oil to overcome the aforesaid existing drawbacks.

### DETAILED DESCRIPTION OF THE INVENTION

This invention provides soft gelatine capsules containing olive oil, hereinafter the inventive capsules, comprising a gelatinous wall and olive oil.

Soft gelatine capsules are containers generally having a spherical, spheroid, oval, oblong or elongate shape, consisting of a single part, are generally elastic and soft, and are made using a mixture of gelatine, water and a plasticiser selected from a group consisting of glycerine, sorbitol, propylene glycol, polyethylene glycol and mixtures thereof, which make up the gelatinous wall, and have a size approximately ranging between 5 mm and 2 cm. Inside the gelatinous wall making up the soft gelatine capsule is the material filling the capsule, specifically duly dosed olive oil, optionally along with other additives or substances. The formulation of the gelatinous wall may also contain suitable additives or excipients, such as opacifiers, colourants (to distinguish capsules designed for certain applications from others), preservatives. etc.

Olive oil present in the inventive capsules as a material filling the capsules, either alone or combined with other additives, may be any commercial olive oil, such as pure and refined olive oil with an acidity of not more than 11 and a peroxide number of less than 15, preferably less than 10).

Administering olive oil in soft gelatine capsules allows the olive oil to be optimally dosed, strictly controlled as to quality, preventing the presence of contaminating impurities, and it is moreover protected from going stale due to contact with air.

Olive oil is compatible with many substances with cosmetic, nutritional or therapeutic applications which may complement the many positive effects of olive oil and which may be incorporated using olive oil as an administration vehicle in the inventive capsules. The inventive capsules may therefore in addition to olive oil, if so desired, contain as a filler material other interesting additives or substances which may add to the beneficial effects of olive oil. The substances which can be added to olive oil as a material filling the inventive capsules are vitamins, trace elements, oils, flavourings, colourants, antioxidants, perfumes, etc.

The inventive capsules may, if so desired, contain as a filler material not only olive oil but a fat-soluble vitamin selected from a group consisting of vitamins A, D, E, F, K and mixtures thereof.

Vitamin A, with a recommended daily quantity (RDQ) of 600 to 700 g (micrograms) [10,000-25,000 IU (international units)] is vital to the daily body functions and is very important for children's growth and development. Vitamin A helps keep the skin healthy, and indeed teeth, bones and membranous mucosae present in the nose and throat, and plays a very important role in maintaining eyesight. It moreover acts as an antioxidant and to protect and foster immunity. The inventive capsules may contain between 400 and 1,200 µg of vitamin A.

Vitamin D, with an RDQ of up to 10 g [200-400 IU] is responsible for absorbing calcium to constitute and maintain the bone tissue and teeth. The inventive capsules may contain between 0.5 and 2.5 g of vitamin D.

Vitamin E, with an RDQ of between 5 and 7 mg (milligrams) [200-800 IU], acts as an antioxidant protecting fatty tissue and cells from premature death or damage and also has an anticarcinogenic function and prevents heart attacks and Alzheimer's disease. The inventive capsule may contain between 0.7 and 0.5 mg of vitamin E.

Vitamin F is necessary for maintaining membrane fluidity, for preserving the elasticity of arterial walls, and for maintaining mucosae, organs and nerves functioning properly. Vitamin F nourishes the skin, maintains blood fluidity, prevents the appearance of bronchial asthma, hypertension, modulates the immune system, rheumatoid arthritis and coronary diseases, maintaining a correct cholesterol level. The inventive capsules may contain between 5 and 100 mg of vitamin F.

Vitamin K, with an RDQ of between 50 and 500 mg is essential to coagulate blood and is therefore ascribed properties in healing injuries. The inventive capsules may contain between 5 and 100 mg of vitamin K.

The addition of said fat-soluble vitamins to the material filling the inventive capsules allows fat-soluble polyvitamin complexes to be developed which may contain one or more of the fat-soluble vitamins (A, D, E, F and K) in the following quantities: vitamin A (400-1,200 µg), vitamin D (0.5-2.5 µg), vitamin E (0.7-5.0 mg), vitamin K (5.0-100 mg) and vitamin F (5.0-100 mg), preferably in quantities within the RDQ range of each of the vitamins present in the complex.

The fat-soluble polyvitamin complex excipient is preferably olive oil which in turn contributes its beneficial components [oleic, linoleic, linolenic and arachidonic acid].

The material filling the inventive capsules may also contain, if so desired, a trace element selected from a group consisting of calcium, copper, chromium, tin, phosphorous, iron, magnesium, manganese, potassium, selenium, silicon, iodine, zinc and mixtures thereof, in the following quantities: calcium (25-50 mg), copper (0.5-2 mg), chromium (2.5-10 µg), tin (2.5-10 µg), phosphorous (25-50 mg), iron (3.5-7 mg), magnesium (25-50 mg), manganese (2.5-10 mg), potassium (2.5-10 mg), selenium (2.5-10 µg), silicon (0.5-2 µg), iodine (25-50 mg) and zinc (3.5-7 mg).

The material filling the inventive capsules may also contain, if so desired, variable quantities of one or several vegetable oils, for instance borage oil, soya oil, sunflower oil, safflower oil, avocado oil, peanut oil and/or one or several animal oils, especially fish oils, for instance oils from fish containing between 0.5 and 5% by weight of 5,8,11,14,17-eicosapentenoic acid [known as omega-3 acid (20:5) or EPA], EPA enriched oils. In a specific embodiment, the material filling the inventive capsule contains between 0.2 and 10% by weight over the total filler material of borage oil.

The inventive capsules may contain, if so desired, conventional antioxidants, preservatives, perfumes and flavourings, suitable for the applications for which they are designed.

Additionally, the inventive capsules may also contain water-soluble additives or substances. To do so, in a specific embodiment, the inventive capsules may be bicompartmentalised, containing olive oil in one compartment, optionally along with other fat-soluble additives or substances, and water-soluble additives or substances in the other compartment, such as water-soluble vitamins for instance, namely B vitamin complex and vitamin C.

In a specific embodiment of the invention, the inventive capsules contain a filler material comprising in addition to olive oil one or several fat-soluble vitamins, and/or one or several of the aforesaid trace elements, and/or one or several of the above-mentioned oils, along with suitable antioxidants, preservatives, perfumes and flavourings. In another specific embodiment of the invention, the inventive capsules contain a filler material comprising in addition to olive oil one or several fat-soluble vitamins and/or one or several of the aforesaid trace elements, and/or one or several of the above-mentioned oils, and/or one or several water-soluble vitamins, optionally along with suitable antioxidants, preservatives perfumes and flavourings.

Generally, the inventive capsules may contain between 10 and 1,200 mg of total filler material consisting of either olive oil on its own or olive oil and other additives.

The inventive capsules constituting a solid form of administering olive oil, expediting its oral ingestion both by people used to the taste and smell of olive oil and people unaccustomed to such organoleptic characteristics, or liable to retch and/or vomit in trying to take olive oil in the conventional manner, by traditional liquid oral means, for the inventive capsules allow the organoleptic characteristics of olive oil to be disguised in such a way that it may be taken without its smell or taste being perceived.

The inventive capsules may be used with cosmetic and/or nutritional and/or therapeutic purposes.

Amongst their cosmetic applications, reference can for instance be made to using the inventive capsules to protect the skin from the appearance of wrinkles and as a reliever for dry and scaly skin. They may also be used to recover the brightness and strength of strained hair and eyebrows and to restore the vitality of frail hair. In applications of this kind, the inventive capsules may be administered either orally (internally) or topically (externally). In the latter case, the inventive capsules are broken and their contents poured onto the application area, for instance skin, scalp or mucosae. This topical application of olive oil for cosmetic purposes can be very interesting in people who are not allergic to fat-soluble vitamins. However, in people who have allergic reactions to contact with the vitamins present in some cosmetics, the oral administration of the inventive capsules containing vitamins is an ideal solution. Applied to the skin, the olive oil contained in the inventive capsules acts as an antioxidant, ageing retardant and skin regenerator. In the hair, the olive oil contained in the inventive capsules acts as a revitalising agent and as an agent suitable for treating seborrhoea, atypical dermatitis and the problem of open tips. In mucosae, olive oil helps treat dry oral mucosae (xerostomia or dry mouth syndrome), treating sores or ulcers protecting ulcerated mucosae from bacterial colonisation, and in mouth care, for it is capable of entraining more lipophilic bacteria than water and alcohol, commonly used in mouthwashes. Furthermore, olive oil poured into the mouth cavity upon the inventive capsules being chewed before eating, helps adapt mouth appliances and prostheses, lubricating the same. Although the soft gelatine capsules are usually spherical, oval or oblong in shape, the inventive capsules may, if so desired and essentially for cosmetic applications, be fancifully shaped.

Amongst the nutritional applications of the inventive capsules, reference can be made to their usefulness in contributing unsaturated fatty (oleic, linoleic, arachidonic) acids and saturated fatty (palmitic, stearic) acids to the organism. Generally, the inventive capsules are orally administered in these applications. In a specific embodiment, the inventive capsules designed for nutritional applications have a total weight of 150 mg and filler material contents ranging between 50% and 75% by weight of the total weight of such capsule. In another specific and preferred embodiment, the invention provides soft gelatine capsules with a total weight of 150 mg containing between 75 and 112.5 mg of olive oil only as filler material.

The therapeutic applications of the inventive capsules include preventing and curing many ailments which nowadays stand as the main causes of mortality in developed countries. Thus, it is beneficial in preventing cardiovascular diseases, defending the mechanisms causing ageing, preventing carcinogenesis, hepatic disorders, inflammation, gastritis and ulcers, decrease of the incidence of cholelithiasis, inducing bone mineralisation developing the brain and nervous system, protecting the body from infections and in healing inner and outer tissue.

Furthermore, it has been found during the tests carried out with the inventive capsules that they are suitable for treating halitosis orally. Halitosis or bad breath is a problem with a serious social connotation which affects many people, and is caused by the production and release of volatile compounds, mainly volatile sulphured derivatives, and depending on where the source of the bad smell is located, it may be oral [located in the lips, tongue, teeth, dental prostheses. periodontal tissue, oropharynx] or not [caused by respiratory system diseases, systemic diseases (hepatic failure, cirrhosis, diabetic ketoacidosis, carcinomas and certain metabolic diseases in which an enzymatic anomaly occurs), diseases of the gastrointestinal tract and certain foods, drinks, tobacco and drugs]. There are several treatments to check halitosis, some of which comprise using a combination of olive oil and parsley oil [Spanish patent application no. 9800228]. It has now been found that the inventive capsules containing olive oil alone or along with parsley oil provide an effective treatment against halitosis.

The inventive capsules can be administered either orally, in which case the material filling the capsules is released in the stomach, or topically, in which case they can be chewed in order to release the filler material in the mouth cavity, or they may be broken with the assistance of a suitable device, such as a plastic or metal punch, and its contents poured onto the place of application, such as the hair, skin or mucosae for instance.

The posology of the inventive capsules depends on many factors, which include their application, the condition of the person, the dosage and formulation of the capsules, the ingestion or otherwise of other vitamin or mineral complexes, etc.

The use of the inventive capsules has many advantages, such as the following:
- simple composition of their contents, olive oil and optionally interesting additives or substances, for little or no excipient is required, which allows the potential incompatibilities which might arise to be properly controlled;
- easy preparation and quick and easy dosage;
- the olive oil contained in the capsule is protected, preventing it from going stale through exposure to external agents (air);
- good organoleptic properties, because they are aesthetically pleasing, may be coloured, have no taste although if so desired may be flavoured, and are easy to ingest for, when in contact with saliva, they become slippery and are easily swallowed;
- versatility, for because their manufacture may be magistral, compositions and dosages may be selected suited to each particular user;
- functional versatility, for they may go directly to the stomach, if swallowed, or may serve their purpose orally if chewed;
- good tolerance and bioavailability, for they are quick to release their contents in the gastric medium;
- their dosage is very accurate;
- they are small in size, which makes them easy to store, carry and administer in any environment and at any time;
- they are convenient and simple to administer; and
- they disguise tastes and smells which may be unpleasant for user.

The inventive capsules may be obtained by any conventional process for manufacturing soft gelatine capsules, such as for instance the roller method, or the dripping system.

The roller method comprises using two horizontally arranged rollers turning in opposite directions, one next to the other, with symmetrically matching capsule-shaped dies dug out therein. Each cylinder is provided with a moderately warm sheet of the gelatinous mass making up the wall of the capsule, which is taken to the place at which the two rollers meet through their movement. A hopper lying over that place intermittently and regularly pours quantities of the liquid [olive oil and optional additives or substances] making up the filling of the capsule, which is usually added slightly warmed up to help soften the gelatinous mass, which adapts exactly to the mould of the cylinders and results in the capsule. The two parts of the capsule are joined at the point of tangency of the cylinders in such a way that the finished capsules leave through the lower part of the cylinders.

The dripping method comprises using two concentric injection tubes, with their nozzles leading into a tank full of cold paraffin oil (51°C approximately). The liquid filling the capsule flows through the internal tube and the warmed gelatinous mass constituting the walls of the soft capsule flows through the outer tube. The simultaneous outlet of both liquids results in the soft gelatine capsules. The resultant capsules are collected in the cold liquid paraffin bath, washed with an organic solvent and dried in a tray heater.

The capsules obtained by any of the methods are duly tested as to uniform weight, uniform contents, breakdown and dissolution.

A discussion of the magistral and industrial methods of preparing soft gelatine capsules may be found in Tratado de Farmacia Galénica, C. Fauli i Trillo, Luzán 5 S.A. de Ediciones, 10th Edition (1993), Chapter XLI, pages 587-592.

The following examples are useful to illustrate the specific ways of embodying the object of the present invention, although they should not be taken to limit the scope of the invention.

### EXAMPLE 1

### Preparing soft gelatine capsules containing olive oil

Soft gelatine capsules containing 200 mg of olive oil per capsule were prepared by encapsulating each composition in soft gelatine capsules, obtained by mixing gelatine and glycerine using conventional techniques [Tratado de Farmacia Galénica, C. Fauli i Trillo, Luzán 5 S.A. de Ediciones, 10th Edition (1993), Chapter XLI, pages 587-592].

### EXAMPLE 2

### Preparing soft gelatine capsules with olive oil and vitamins suitable for the mouth cavity

Bicompartmentalised soft gelatine capsules containing 300 mg of a filler material based on several quantities of olive oil and fat-soluble and water-soluble vitamins per capsule were prepared. Conventional techniques were used in so doing to fill one of the compartments with olive oil, 0.5-1% of vitamin A, and 0.5-1% of vitamin E, and the other compartment with 0-5-1% of vitamin B5 and 2.5-10% of vitamin C, colourants and flavourings (in sufficient and adequate quantities).

The capsules obtained are suitable to be topically administered in the oral cavity by breaking the same. The filler material allows the mucosae to be humidified, teeth and mucosae to be cleaned and microorganisms involved in halitosis to be checked.

### EXAMPLE 3

### Preparing soft gelatine capsules with olive oil and vitamins suitable for the hair and skin

Soft gelatine capsules containing 300 mg of a filler material comprising olive oil, fat-soluble and water-soluble vitamins, oils and vegetable proteins, per capsule, were prepared. Specifically, the filler material comprised olive oil, 2.5% of vitamin C, 5% of silk protein, 5% of nut oils, 3% of essential oils, 3% of paraffin, 0.5% of polyethylene glycol and 0.3% of castor oil, colourants and perfumes in sufficient quantities.

The capsules obtained are suitable to be topically administered on the skin and hair in order to foster body care.

### EXAMPLE 4

### Preparing soft gelatine capsules with olive oil suitable for checking halitosis orally

Soft gelatine capsules containing 200 mg of a filler material comprising variable quantities of olive oil, parsley oil, mint oil, chlorophyll, and menthol, per capsule, were prepared. Specifically, the filler material consisted of a composition comprising 54-89% by weight of olive oil. 5-20% by weight of parsley oil, 5-20% by weight of mint oil, 1-5% by weight of chlorophyll and 0-1% by weight of menthol.

These capsules are suitable for checking halitosis.

### EXAMPLE 5

### Preparing soft gelatine capsules with olive oil suitable for improving blood clotting and for preventing cardiovascular diseases

In this case, soft gelatine capsules containing variable quantities of a filler material containing olive oil along with other oils selected from a group consisting of borage oil, fish oils with EPA, soya oil, sunflower oil, safflower oil, avocado oil, peanut oil and mixtures thereof, were prepared.

## Claims

1. A soft gelatine capsule containing olive oil, which comprises a gelatinous wall and olive oil.

2. A capsule in accordance with claim 1, wherein said gelatinous wall comprises a mixture of gelatine, water and a plasticiser.

3. A capsule in accordance with claim 2, wherein said plasticiser is selected from a group consisting of glycerine, sorbitol, propylene glycol, polyethylene glycol and mixtures thereof.

4. A capsule in accordance with claim 2, wherein said gelatinous wall moreover comprises opacifiers, colourants and preservatives.

5. A capsule in accordance with claim 1, wherein said olive oil is a commercial olive oil.

6. A capsule in accordance with claim 1, wherein said olive oil is selected from a group consisting of pure olive oil, refined olive oil and mixtures thereof.

7. A capsule in accordance with claim 6, wherein said olive oil comprises a mixture of pure and refined olive oils with an acidity of not more than 11 and a peroxide number of less than 15.

8. A capsule in accordance with claim 1, further comprising additives or substances selected from a group consisting of vitamins, trace elements, oils, flavourings, colourants, antioxidants, perfumes and mixtures thereof.

9. A capsule in accordance with claim 8, wherein said vitamins are fat-soluble vitamins selected from a group consisting of vitamins A, D, E, F. K and mixtures thereof.

10. A capsule in accordance with claim 8, wherein said trace elements are selected from a group consisting of calcium, copper, chromium, tin, phosphorous, iron, magnesium, manganese, potassium, selenium, silicon, iodine, zinc and mixtures thereof.

11. A capsule in accordance with claim 8, wherein said oils are selected from a group consisting of vegetable oils, animal oils and mixtures thereof.

12. A capsule in accordance with claim 1, provided with two compartments, one of which compartments is to hold the olive oil, optionally along with other fat-soluble additives or substances, and the other compartment is to hold water-soluble additives or substances.

13. A capsule in accordance with claim 12, which comprises at least a water-soluble vitamin.
